# EUROPEAN PATENT APPLICATION

(11) **EP 3 859 316 A1**
(43) Date of publication of application: **04.08.2021**
(21) Application number: 20155026.6
(22) Date of filing: 31.01.2020
(51) Int. Cl.: G01N 21/77, G01N 21/78, B01L 3/00, G01N 33/558

(54) **TESTING DEVICE FOR LATERAL FLOW ASSAY**

(71) Applicant: Midge Medical GmbH, 12103 Berlin (DE)
(72) Inventor: RIESTER, Markus, Dr., Wiesbaden, Hessen (DE); DIEBOLD, Michael, Berlin, Berlin (DE)
(74) Representative: Eisenführ Speiser

(57) **Abstract**

A testing device (160) with a testing assembly (170) for lateral flow assay is disclosed. The testing assembly (170) comprises liquid sample receiving interface (106) arranged on a support structure (104) defining a plane. The liquid sample receiving interface (106) is configured to receive a liquid sample. The testing assembly (170) comprises at least one testing strip (108.1, 108.2) fluidly connected to the liquid sample receiving interface (106). The testing strip (108.1, 108.2) comprises a capillary wick (110.1) fluidly connected to the liquid sample receiving interface (106) and including at least one test portion (112.1, 112.2), the test portion (112.1, 112.2) comprising at least one respective reacting material configured for reacting in a predetermined manner to at least one specific analyte. The testing device (160) comprises an optical sensor (111), arranged and configured for detecting light reflected from the at least one test portion (112.1, 112.2) and for converting the detected light into an electrical signal representing an intensity and/or a color of the detected light. The testing device (160) further comprises a conversion unit (116) for converting the electrical signal into digital data representing the intensity and/or the color of the detected light, and a transmitter unit (113) for wirelessly transmitting digital data.

## Description

### FIELD OF THE INVENTION

The present invention relates to a testing device for lateral flow assay.

### BACKGROUND OF THE INVENTION

Lateral flow assays, also known as lateral flow immunochromatographic assays, are devices for detecting the presence (or absence) of a target analyte in a sample. Typically, these tests are used for medical diagnostics either for home testing, point of care testing, or laboratory use. The technology is based on a series of capillary beds having the capacity to transport fluid spontaneously by, for example, capillarity effect.

WO 2019/145374 describes a testing assembly for lateral flow assay which comprises a liquid sample receiving interface configured to receive a liquid sample and at least one testing strip fluidly connected to the liquid sample receiving interface and comprising a capillary wick also fluidly connected to the liquid sample receiving interface and including at least one test portion. The test portion comprises at least one reacting material configured to react in a predetermined manner to at least one pre-specified analyte.

### SUMMARY OF THE INVENTION

It is an object to provide an improved testing device for lateral flow assay.

According to the invention the object is achieved by a testing device comprising a testing assembly. The testing assembly comprises a liquid sample receiving interface configured to receive a liquid sample. The liquid sample receiving interface is arranged on a support structure that defines a plane. The testing assembly comprises at least one testing strip that is fluidly connected to the liquid sample receiving interface. The testing strip includes a capillary wick fluidly connected to the liquid sample receiving interface. The capillary wick includes at least one test portion. The test portion comprises at least one reacting material configured to react in a predetermined manner to at least one specific analyte.

The testing device further comprises an optical sensor, a conversion unit and a transmitter unit. The optical sensor is configured for responding to impeding light and for providing an electrical signal representing a property, e.g., intensity or color or polarization, of the impeding light. The conversion unit is part of or operatively connected to the optical sensor and configured for converting the electrical signal into digital data representing the electrical signal and the transmitter unit is configured for wirelessly transmitting digital data.

The components and sub-components of the testing device are as follows:
The testing device comprises
- a testing assembly,
- an optical sensor,
- a conversion unit, and
- a transmitter unit.

The testing assembly comprises
- a support structure,
- a sample receiving interface, and
- at least one testing strip, wherein the testing strip comprises
   - a capillary wick, and wherein the capillary wick includes
   - at least one test portion.

The optical sensor, the conversion unit and the transmitter unit can be arranged on the testing assembly's support structure. Alternatively, the optical sensor, the conversion unit and the transmitter unit can be attached to a testing device's cover unit.

The testing device's optical sensor is arranged and configured for detecting light reflected from the at least one test portion, and for providing an electrical signal representing an intensity and/or a color of the detected light. The optical sensor can be, e.g., a single-pixel photodiode or a CMOS-sensor or a CCD-sensor.

After converting detected light into an electrical signal, the electrical signal is provided to the conversion unit.

The conversion unit is configured for converting the electrical signal into digital data representing the intensity and/or the color of the detected light. The conversion unit can be part of the optical sensor. The conversion unit can also be a separate component of the testing device. Alternatively, the conversion unit can be part of the transmitter unit. Preferably, the conversion unit is or comprises an analog-to-digital converter (ADC) for converting the electrical signal into digital data representing the intensity and/or the color of the detected light. The conversion unit can be configured for converting the electrical signal with 8-bit.

The conversion unit is operatively connected to the transmitter unit, e.g., via a data bus, for providing digital data to the transmitter unit.

The transmitter unit is configured for wirelessly transmitting the digital data, preferably, to an external receiving device using a predetermined wireless communication protocol such as Bluetooth, near-field communication (NFC) or Wi-Fi or RFID. In particular, the transmitter unit can be or can comprise a NFC-chip and a NFC-coil, or a radio-frequency identification (RFID)-tag or transponder, or a Wi-Fi-integrated circuit chip, or a Bluetooth integrated circuit chip.

A transmitter unit based on a technology such as NFC or RFID not requiring a permanent energy supply is preferred. The energy needed for powering such a transmitter unit is provided by a so-called initiator.

A transmitter unit that is configured for transmitting data via NFC or RFID, preferably, comprises one or more antennas functioning as a radio-frequency (RF) interface for transmitting electromagnetic signals representing digital data by means of electromagnetic induction to one or more further antennas of an external device. Antennas typically comprise one or more coils each having 4 or 5 windings.

The initiator can be the external device providing a carrier field that is modulated by the transmitter unit for transmitting digital data. Preferably, for powering the transmitter unit, the transmitter unit draws energy from the external device via the NFC or RFID link. Thus, in particular, in case the transmitter unit is NFC- or RFID-enabled, it is not necessary that the testing device itself comprises an energy storage unit, e.g., a battery, for powering the transmitter unit.

The testing device according to the invention is a single device allowing receiving a liquid sample, e.g., a body fluid such as blood, from a patient, processing the received liquid sample with a microfluidic system comprising a capillary wick with at least one test portion and analyzing the received body fluid with respect to the presence of a specific analyte. The evaluation whether or not a specific analyte is present in the liquid sample is performed externally, e.g., directly on an external device that receives the digital data. The external device can be a smartphone, or a tablet, preferably, having NFC or RFID capabilities and being configured to function as an initiator device. The external device can also be used to transmit the digital data further, e.g., to a personal computer or a server for evaluation purposes.

Preferably, the liquid sample receiving unit is dimensioned and configured to be connected to a piercing element, such as a lancet or a needle, for extracting the liquid sample, e.g. from a body or a container and transporting the liquid sample to the liquid sample receiving unit.

The optical sensor, the conversion unit and the transmitter unit can be arranged as separate components on the support structure together with the microfluidic components. It is also possible that the optical sensor, the conversion unit and the transmitter unit are be fabricated using electronic packaging, e.g., 3D-packaging. Using 3D-packaging, thus, by stacking the components on top of each other, a compact three-dimensional integrated circuit can be designed. After 3D-packaging the integrated circuit comprising the optical sensor, the conversion unit and the transmitter unit can be mounted onto the support structure or attached to a cover unit. Alternatively, a chip comprising the optical sensor, the conversion unit and the transmitter can be fabricated using wafer-level packaging (WLP). The optical sensor, the conversion unit and the transmitter unit can also be put into a protective package for integration into the testing device.

In some embodiments, the optical sensor, the conversion unit and the transmitter unit are mounted on a circuit board that is attached as a module onto the support structure or to a cover unit. The circuit board can be flexible, e.g., a flexible substrate made of polyimide, e.g., Kapton, polyether ether ketone (PEEK), liquid-crystal polymer (LCP), or FR4. Also a rigid or semi-flex circuit board can be used as an alternative. In particular, the optical sensor, the conversion unit and the transmitter can be integrated onto a thin FR4 substrate. The circuit board can be a printed circuit board (PCB) which, preferably, is flexible, e.g., a FR4 PCB. Alternatively, the printed circuit board can be a rigid or semi-rigid printed circuit board.

Attaching at least of the optical sensor, the conversion unit and the transmitter unit to a testing device's cover unit is of advantage since more space is left on the support structure, e.g., for arranging the components of the microfluidic system.

In particular, an antenna of the transmitter unit can be integrated in the testing device using in-mold manufacturing. In case the testing device has a cover unit attached to the support structure, thus, forming a closed housing, an antenna of the transmitter can be integrated into the housing, e.g., attached on the inside to the lid part of the cover unit by means of in-mold manufacturing. Alternatively, an antenna of the transmitter unit can be integrated into the same chip or in the same circuit as the remaining transmitter electronics, the optical sensor and the conversion unit. For instance, the antenna can be integrated in the PCB.

Preferably, the testing device comprises an optical arrangement comprising one or more optical elements that are arranged and configured for directing light reflected from the at least one test portion to the optical sensor and/or for directing light emitted from a light source to the at least one test portion. An optical element can be a mirror or a lens or a waveguide. The optical elements serve for creating an optical path linking, e.g., the at least one test portion of the testing strip to the optical sensor.

For example, a mirror can be used for directing light reflected from the test portion and a lens can be employed for focusing the light reflected from the mirror onto the optical sensor. It is also possible that a waveguide is used for directing the light reflected from the at least one test portion to the optical sensor. The waveguide can be shaped at its one end for focusing light onto the optical sensor.

An optical arrangement is of advantage, in particular, if the test portion and the optical sensor are not aligned, i.e., the test portion is not in the immediate field of view of the optical sensor.

An optical arrangement is also of particular advantage if a test strip comprises more than one test portions, e.g., three test portions. In this case, an optical element of the optical arrangement, preferably, is configured and arranged for directing light reflected from anyone of the three test portions to the optical sensor. An accordingly configured optical element can comprise three mirror surfaces that are inclined to each other in a way that light reflected from anyone of the test portions is directed to the optical sensor upon reflection on one of the three inclined mirror surfaces. Thus, in general, an optical element can comprise a plurality of reflecting surfaces, e.g., a plurality of facets, that are configured and arranged for directing light reflected from one or more test portions of a plurality of test portions that are arranged along the length of one testing strip to the optical sensor. An external receiving device can receive the digital data from the transmitter unit wherein the digital data representing light intensity and/or color of the light reflected from one or more of the plurality of test portions of the testing strip.

In general, using an optical arrangement allows to more freely arrange the test strip and the optical sensor in the testing device, e.g., on the support structure since light paths can be created connecting test portion and optical sensor. Such light paths can have an angle or can be curved, e.g., when using waveguides.

If the testing device comprises a cover unit, the optical sensor can be mounted to a cover unit. Such a cover unit, preferably, is configured to be permanently, or releasably attached to the support structure to form a closed housing together with the support structure. To ensure correct alignment of the at least one test portion and the optical sensor it is of advantage if the cover unit can only be attached to the support structure in one or more distinct positions.

Inside the housing, preferably, all components of the testing device, i.e., the microfluidic system comprising the test strip, and the optical sensor, the conversion unit and the transmitter unit are accommodated.

Preferably, the surface of the of the testing device is small in relation to the volume and smooth with as little gaps as possible to thus reduce the risk of infestation.

Preferably, the testing device with a cover unit attached to the support structure has a cylindrical shape and a height that is equal to or smaller than 3 cm, preferably smaller than 2 cm, more preferably smaller than 1.5 cm, even more preferably equal to or smaller than 1 cm, and a diameter that is equal to or smaller than 5 cm, preferably smaller than 4 cm, more preferably smaller than 3 cm, even more preferably equal to or smaller than 2.5 cm.

Alternatively, the testing device can have a cubic or rectangular base area with sides having a length that are equal to or smaller than 5 cm, preferably smaller than 4 cm, more preferably smaller than 3 cm, even more preferably equal to or smaller than 2.5 cm, the testing device having a height that is equal to or smaller than 3 cm, preferably smaller than 2 cm, more preferably smaller than 1.5 cm, even more preferably equal to or smaller than 1 cm.

It is an advantage that the testing device can be designed as a comparatively small and compact device. In case the testing device has a cover unit attached to the support structure, cover unit and support structure preferably form a small and compact housing having a closed outer surface except of the sample receiving interface. Preferably, no push buttons or other movable parts are integrated in the housing wall. Preferably, also no windows or displays are integrated in the housing wall. The cover unit's walls defining the cover unit's outer surface, however, can be deflectable to allow manual actuation of mechanical and/or electrical components inside the testing device.

The testing device thus is a closed device being able to communicate with an external device via the transmitter unit. As soon as a liquid sample is received by the sample receiving interface no further user interaction is required for starting processing and analysing the liquid sample.

The testing device, preferably, has a comparatively small outer surface in relation to the testing device's volume. Preferably, the ratio S/V between outer surface S and volume V is between 6.0 and 0.8 [1/cm], more preferably, between 5.0 and 1.0 [1/cm], even more preferably between 4.5 and 1.2 [1/cm], even more preferably between 4.0 and 1.4 [1/cm].

In embodiments where the testing device has a cover unit, the optical sensor, preferably, is arranged facing the support structure. In an embodiment in which the test strip and, in particular, the test strip's one or more test portions face a testing device's sidewall, i.e., being arranged at an angle of 90° with respect to the optical sensor, an optical element of the optical arrangement, preferably, is configured and arranged to redirect light reflected from the one or more test portions about 90° towards the optical sensor, i.e., vertically with respect to the support structure. Thus, in some embodiments of the testing device the at least one test portion and the optical sensor are arranged at an angle of 90° with respect to each other and an optical element is arranged and configured for redirecting light reflected from the at least one test portion at an angle of 90° from the at least one test portion to the optical sensor.

The testing device can optionally comprise at least one light source that is arranged and configured to illuminate the at least one test portion. The one or more light sources are provided as part of the testing device to illuminate the at least one test portion. The light source, preferably, comprises a light emitting diode (LED). The light source, preferably, is connected to an energy storage unit and/or an optional power management unit. In case the transmitter unit is configured to draw energy from an external device, the one or more light source can also be powered with energy drawn from the external device while the NFC or RFID connection is established.

Preferably, in a testing device having a light source, the light source is arranged to emit light towards the at least one test portion. The light is reflected at the test portion and detected with the optical sensor. An optical arrangement comprising a number of optical elements can be used to redirect the light emitted from light source to the at least one test portion and/or to redirect the light reflected from the at least one test portion to the optical sensor. Thus, by means of the optical arrangement light paths linking the light source and the test portion and/or the test portion and the optical sensor can be created. For example, a waveguide can be used to guide light emitted from the light source to the test portion and a mirror can be used to redirect light reflected from the test portion to the optical sensor, that, e.g., is attached to a testing device's cover unit. Accordingly, the testing device can comprise an optical arrangement comprising one or more optical elements that are arranged and configured for directing illuminating light emitted by the at least one light source to the at least one test portion.

Preferably, the light source used for illuminating the at least one test portion is configured for emitting light at a color corresponding to the color that the test portion takes in case a specific analyte is present in a received liquid sample. A change of the test portion's color into the color of the light emitted by the light source yields an increase in the intensity of light reflected from the test portion. By means of the intensity it is thus possible to find out whether or not a specific analyte is present in a liquid sample.

Preferably, prior to using of the testing device or as part of manufacturing process of the testing device, the optical sensor is calibrated. Calibration can comprise sensing the light intensity reflected from the unused test strip when the light source is switched on and when the light source is switched off to thus determine a maximum and a minimum of the intensity that can occur. A light intensity resulting from the test portion after being exposed to an analyte can therefore be evaluated more precisely. Calibration data can be stored, e.g., on a server or on a local memory comprised in the testing device that can be accessed by an external device via NFC or RFID. For example, calibration data can be transmitted while a NFC or RFID link is established between the testing device and an external device and used, e.g., by the testing device for evaluation purposes.

In case the testing strip comprises more than one test portion, e.g., two or three test portions or even a test portion matrix, a single light source can be used to illuminate all test portions present on the test strip. The light intensity of the light reflected from the plurality of test portions depends on whether or not a specific analyte is present in the liquid sample. Using a trained neural network, digital data signals representing the digital data can be analyzed to determine which of the plurality of test portions has changed its color due to presence of a specific analyte.

It is preferred that if the testing strip comprises more than one test portion on a test strip, i.e., at least two test portions, each of the test portions is configured to react to a different one of various analytes. Preferably, the color that one of the test portions takes in case a specific analyte is present in a received sample liquid is different to the colors that the other test portions take in case another analyte is present in a received sample liquid. For example, the test strip can comprise a test portion matrix comprising a plurality of test portions, each being configured for indicating the presence of a different one of various analytes. Such a test portion matrix can be, e.g., a four-by-four test portion matrix or a five-by-five test portion matrix.

For each of a plurality of a test strip's test portions, a light source can be present, preferably, in a one-to-one assignment between one of the test portions and one of the light sources. Preferably, a light source assigned to one of the test portions is configured to emit light in a color that corresponds to the color that the respective test portion takes in case a specific analyte is present in a received liquid sample. For each of the light sources, a lens can be provided that is arranged and configured for focusing light emitted by the respective light source onto the respectively assigned test portion. It is also possible that for each of the light sources a waveguide is provided that is arranged and configured for guiding light emitted from the respective light sources to their assigned test portions.

Preferably, in case more than one light source is present, the at least two light sources can be controlled independently, i.e., can be switched on and off independently of each other by controlling switches. To this end, the testing device can comprise a microcontroller that can receive control commands from an external device via an established NFC or RFID link. Thereby, also a spatial resolution of the at least two test portions can be achieved.

If the test strip comprises more than one test portion, it can be advantageous for determining the colors of the test portions if two or more independently controllable light sources are provided, each emitting light in a different color. For example, three LEDs, one emitting red light, one emitting green light, and one emitting blue light can be used. With each of the LEDs, the plurality of test portions can sequentially be illuminated to capture the intensity of reflected red, green or blue light that is reflected from the plurality of test portions. The captured intensity values can be combined to generate a color value for a respective test portion. If for each illumination an intensity image (grey scale image) is recoded, a color image can be generated from the intensity images. The external device also can be configured to process the intensity images separately, each intensity image represented by image data. The image data can be analyzed by a trained neural network that is fed with a color matrix or with a combined intensity matrix. In an alternative embodiment of the neural network, the neural network can be trained to classify digital data representing electrical signal provided by the single-pixel of a single-pixel optical sensor. The classifying neural network in these embodiments can provide an instant test result. The trained neural network may be implemented within the testing device by means of a processing unit and a memory connected thereto and software. Alternatively, or additionally, a trained neural network can be part of an external device that is configured to communicate with the testing device.

The light reflected from the at least two test portions is reflected to and detected by the optical sensor. An optical element can be present that is arranged and configured for redirecting light reflected from the at least two test portions to the optical sensor. In case, the light sources are switched on and off sequentially by controlling switches, each of the test portions can be read out separately one after another. The optical sensor can thus be used to sequentially detect impeding light reflected by the at least two test portions and to sequentially convert light reflected from one of the test portions into an electrical signal representing intensity and/or color of the light reflected by one of the test portions. Thus, by switching the light sources a particular test portion can be selected and read out.

The one or more light sources preferably are arranged such that the test portions of the testing assembly are illuminated by the one or more light sources. For example, each test portion can be illuminated with a different one of various LED's in a one-to-one assignment. The light sources can be attached to a cover unit.

The one or more light sources can be used in combination with the optical sensor to determine a filling level of a solution chamber that is optionally comprised by the testing assembly, the solution chamber containing a buffer solution. Alternatively, or additionally, the one or more light sources can be used in combination with the optical sensor to obtain a degree ofwetting of a capillary wick, e.g., for checking whether an amount of supplied liquid sample is sufficient.

By means of the one or more light sources and/or the optical sensor a timer function can be implemented.

The timer function can comprise that the receiving of a liquid sample is detected and a flag is stored together with a time stamp in the transmitter unit indicating the time of activation of the testing device. Thus, an external device can read out the flag and only start retrieving digital data from the testing device after a predetermined time duration has passed. It is also possible that the time of calibration of the testing device is stored together with a flag in the transmitter unit. An external device can read out the flag and retrieve digital data from the testing device only in case the current instant of time lies within a specific time duration that is chosen such that the calibration is expected to remain accurate within this period. Also a flag can be stored together with a time stamp providing the time of manufacturing the testing device such that a testing device reading out the flag and retrieves digital data from the testing device only in case the testing device has not reached its shelf life.

The transmitter unit is configured to have energy harvesting capabilities and thus for receiving energy from an external initiator device wirelessly, e.g., via electromagnetic induction, during transmission. This can be achieved with a transmitter unit for near-field communication. In particular, if the transmitter unit has energy harvesting capabilities, no further voltage and/or current supply, e.g., a battery, is needed in the testing device.

An external device can retrieve the digital data from received electromagnetic signals and can store and/or further process and/or directly visualize retrieved digital data on a monitor. In addition, other receiving devices, that do not act as the external initiator device but are within range, can also receive the digital data.

In particular, if the testing device comprises a transmitter unit with energy harvesting capabilities, the testing device, preferably, comprises a power management unit (PMU) that is configured for supplying received electrical power for powering electronic and/or electro-mechanical components of the testing device. Preferably, the power management unit comprises voltage stabilizing circuitry, in particular a capacitor.

The power management unit can be part of the transmitter unit and can be operatively connected to its antenna, e.g., its NFC-coil. The power management unit can also be a separate component of the testing device and operatively connected to the transmitter unit for harvesting energy.

Optionally, the power management unit can comprise an energy storage unit, e.g., a primary or a secondary electric battery or a capacitor, in particular, a supercapacitor, for storing energy drawn from an external device during transmission. Thus, via an established NFC or RFID link, energy can be transferred from an external initiator device by means of inductive coupling to the testing device. Such an external initiator device thus likewise comprises an NFC-chip and an NFC-coil for transmitting a carrier signal to the testing device's NFC-coil.

A transmitter unit supporting energy harvesting can be used for powering and/or controlling further electronic and/or electro-mechanical components, e.g., a microcontroller, a sensor or a valve or a micro pump or an actuator, of the testing device. Such a sensor can be configured to implement polymerase chain reaction (PCR), e.g., for replicating DNA of a virus in order to detect and classify the DNA of a virus.

If the transmitter unit supports energy harvesting it can operate without a battery by drawing power from an external device via an established NFC or RFID link. A NFC or RFID link typically operates over a distance of several centimeters, e.g., up to 5 cm, or up to 10 cm or up to 20 cm and sometimes even up to, e.g., 60 cm. Via a NFC or RFID link, energy harvesting of up to, e.g., 30 mW can be achieved.

With the testing device it is possible to obtain various operating state information while using the testing device. For example, the testing device can comprise a digital signal processor for analyzing a digital data signal representing an intensity and/or color of the reflected from the at least one test portion with respect to whether blood extraction was successful, a buffer solution was supplied, the test portion's reacting material has reacted with an analyte, or whether light intensity was sufficient. Further sensors can be present in the testing device for sensing the temperature, the pressure or the humidity and for providing respective temperature data, pressure data or humidity data via an NFC link to an external initiator device.

The transmitter unit can be part of a transceiver unit that is configured for transmitting digital data and for receiving control commands. Such transceiver is useful in case the testing device comprises further components such as a microcontroller, a sensor or a valve or a micro pump or an actuator and/or other electromechanical components.

Preferably, the transmitter unit and/or the transceiver unit and, in particular, their NFC-chip comprises a data bus interface, e.g., an I²C interface. Via the data bus interface, the transmitter unit or the transceiver unit can be connected, e.g., to the conversion unit and/or to a microcontroller by way of a data bus.

Preferably, the transmitter unit and/or the transceiver unit and, in particular, their NFC-chip comprises a memory unit. The memory unit can comprise at least one of a volatile memory (such as a static random-access memory (SRAM)) and a non-volatile memory such as an erasable programmable read-only memory (EPROM), in particular, an electrically erasable programmable read-only memory (EEPROM). In the memory unit and, in particular, in the EEPROM, control commands for controlling electronic and/or electro-mechanical components of the testing device can be stored.

A non-volatile memory can also be used for storing digital data, e.g., representing an intensity and/or a color of light detected by the optical sensor. Since from the intensity and/or the color of the light it can be derived whether or not a specific analyte is present in a liquid sample, the digital data can represent confidential patient information that may be linked to personal data or identifiers. Therefore, in particular in case immediate read out of the digital data is not possible at the time of digital data generation, in a preferred embodiment the digital data is at least temporarily stored in a secure memory such as the non-volatile memory of an NFC-chip.

Preferably, the transmitter unit and/or the transceiver unit and, in particular, their NFC-chip comprises a digital control unit (DCU) having at least one of an I²C controller, a pulse-with-modulation (PWM) controller, a general-purpose I/O (GPIO), a command interpreter, and a memory controller.

Preferably, the transmitter unit and/or the transceiver unit and, in particular, their NFC-chip comprises at least one IO terminal pin for connecting an electronic and/or electro-mechanical component of the testing device.

The transceiver unit can receive control commands from an external device for controlling one or more optionally comprised electro-mechanical components of the testing device such as a valve for controlling the amount of buffer solution fed into a solution chamber or a micro pump for pumping body fluid to the test portion of the at least one test strip. For controlling one or more optionally comprised electronic and/or electro-mechanical components, these components can be connected to the transmitter unit's data bus or - for individually addressing one of the electro-mechanical components - to the transmitter unit's IO pins.

Via a data bus, the transceiver unit can also be connected to a microcontroller that is configured for controlling one or more light sources and/or for controlling and/or electronic and/or electro-mechanical components of the testing device. With a microcontroller it is possible to control electronic and/or electro-mechanical components of the testing device in real-time by way of control commands received by the transceiver unit. Such a microcontroller can be powered with energy drawn from an external device or with energy stored in an energy storage unit of a power management unit.

Through the testing assembly's liquid sample receiving interface, an external liquid sample can be transferred along the capillary wick to the one or more testing strips, which are fluidly connected to the liquid sample receiving interface. In this way, at least part of the liquid sample can be transferred via the capillary wick to its at least one testing portion. Thus, the capillary wick has at least one transport portion and at least one test portion, wherein a liquid sample is transported along the capillary wick's transport portion to its test portion.

The test portion of the testing strip acts as a test unit forthe liquid sample, where a presence or an absence of a specific analyte is determined. When exposed to a liquid sample, e.g., body fluid, the test portion is configured for indicating whether or not the at least one specific analyte is present in the liquid sample. Preferably, the presence of a specific analyte within the liquid sample is indicated by the respective test portion by means of a change of the test portion's color. Preferably, the color of the respective test portion indicating the presence a specific analyte is known beforehand and thus can be compared to a reference color for evaluation purposes.

This is possible since the test portion's reacting material reacts only with the liquid sample if it contains the at least one specific analyte. For evaluation purposes the test portion has to be inspected. Typically, inspecting the test portion is done directly by eye which often includes that the color of the test portion is compared by a user to various reference colors to find a match.

The testing device according to the invention makes directly inspecting the test portion by eye obsolete. In particular, it is not necessary that the test portion can actually be seen directly by eye from outside the testing device. Thus, lenses and/or windows for directly inspecting the test portion by eye are not needed. This allows more freely designing the testing device, in particular, in a comparatively compact and small manner. For example, the testing device can have a size that is too small for conveniently inspecting the test portion by eye. Further, the arrangement of the testing strips in the testing assembly is not limited by the need to provide a window for inspecting the test strip's test portion by eye.

The digital data representing an intensity and/or a color of the light that is reflected from the test portion can be transmitted to an external initiator device and persistently stored on its storage medium and/or visualized on a monitor of the external device or a monitor connected to the external device. Therefore, the current state of the test portion at the time of light reflection can be repeatedly analyzed also at a later instant of time using various different visualization capabilities.

The digital data can be processed using, e.g., a digital signal processor, of an external device. For example, the digital data can be processed for evaluating whether or not the at least one specific analyte is present in a provided liquid sample. Also, the digital data can be simultaneously transmitted to a plurality of different external devices for visualization and/or evaluation purposes.

Errors or uncertainties in inspecting the test portion, e.g., originating from the perspective of a user or the light conditions can be avoided with the testing device since an light reflected from the test portion can be detected under constant environmental conditions that can be optimized for the requirements of the optical sensor.

The testing device can optionally comprise a storage medium for storing the digital data prior to transmitting the digital data by means of the transmitter unit.

The testing device can be part of a testing system comprising the testing device and an external device. The testing device is configured to wirelessly transmit the digital data and the external device is configured for receiving the digital data. Thus, testing device and external device have compatible data interfaces and communication means for exchanging digital data and/or control commands.

The testing system can further comprise a server that is operative connected to the external device for transmitting the digital data processed or non-processed from the external device to the server for storage and/or evaluation purposes.

Preferably, the testing strip has, in a planar state, a testing strip center line length, a testing strip width and a testing strip thickness. The testing strip, preferably, has two flat sides that are spaced apart by the testing strip's thickness. The testing strip thickness can have an extension that is shorter than the testing strip center line length and also shorter than the testing strip width.

In particular, if the testing strip is rectangular (neglecting its thickness) in its planar state, the testing strip, preferably, has a testing strip center line length in a longitudinal direction, a testing strip width in a width direction perpendicular to the longitudinal direction and a testing strip thickness in a thickness direction perpendicular to both the longitudinal direction and the width direction, that is shorter, i.e., has a smaller extension, than the testing strip center line length and the testing strip width. In particular, in case of a rectangular testing strip it is preferred that a length of the testing strip and a length of a center line in the middle of the testing strip, hereinafter also referred to as testing strip center line length, is equal to a length of the longitudinal edges of the testing strip's flat surfaces.

Alternatively, the edges of the flat sides can be curved, (i.e. not straight) in the testing strip's planar state. This results in a testing strip having a curved shape in its planar state. In this case the testing strip center line length is the length of a center line located midway between the longitudinal edges of the testing strip. The testing strip center line length is inherent to the testing strip and independent on an actual state - curved or planar - of the testing strip.

The distance between the longitudinal ends of a planar, curved testing strip can be shorter than the length of the center line.

To further limit the outer dimensions of the testing strip - and thus the envelop of the testing strip - the testing strip can be arranged non-planar, i.e., bent or further curved in a third dimension. This includes, for example, testing strips having straight longitudinal edges that are arranged in a curved state, e.g., rectangular testing strips that are folded, curled or wrapped, testing strips with curved longitudinal edges in a flat state, or testing strips with curved longitudinal edges that are folded curled or wrapped and thus in a curved state.

In the testing assembly, a width direction of the testing strip, preferably, extends at an angle smaller than 90° with respect to a normal of the plane defined by the support structure, i.e., the testing strip extends from the support structure. Also, the testing strip, preferably, is curved, resulting in a shortest distance between two opposite longitudinal ends of a testing strip center line being shorter than the testing strip center line length in the planar state. The shortest distance is herein defined as a length amount indicative of a minimum distance amount between a proximal end of the testing strip, i.e., a section of the testing strip being in contact with or in the vicinity of the liquid sample receiving unit, and a distal end of the testing strip at which, or close to which, the test portion is arranged.

It is noted that a shortest distance between the longitudinal ends of the testing strip shorter than a center line length implies that the testing strip is curved, either in its planar state, or because the testing strip is arranged non-planar or both. Testing strips wherein the shortest distance between the longitudinal ends is shorter than its center line length have an effective total extension or envelope of the testing strip that is smaller than the testing strip center line length in the planar state. This, in turn, allows for a size reduction of the testing assembly in comparison with a minimum size that the assembly would have in case the testing strips were arranged in the planar state. If the testing strip is curved in a circular shape, the shorter distance between the longitudinal ends can be shorter than the maximum outer dimension of the testing strip while the maximum outer dimension of the testing strip is still smaller than the testing strip center line.

This advantageous spatial arrangement of the testing strips in the testing assembly enables an improved usage of space within the testing assembly. It further enables a reduction of a total size of the testing assembly without a need to reduce the testing strip center line length. This, in turn, results in an improved applicability and offers an increased versatility.

By arranging the testing strips in a curved way and in a way in which the width direction of the testing strip extends at an angle smaller than 90° with respect to the normal of the plane defined by the support structure, i.e., the testing strips not being arranged parallel to the plane defined by the support structure, the size of the testing assembly can be reduced compared to typical testing assembly configurations, wherein the test strips are usually directly arranged on a support structure in the planar state. Alternatively, longer testing strips can be used when compared to known testing assemblies wherein testing strips are arranged in the planar state onto the support structure.

Preferably, the test portion comprises at least one reacting material configured to react in a pre-specified manner to at least one specific analyte.

Each or at least some of the testing strips can comprise a test portion that is configured to react in a pre-specified manner to a different one of various specific analytes. Alternatively, two or more of the testing strips can have one or more test portions having a given reacting material with a same or a respective different sensitivity, in order to either improve the accuracy of testing assembly or in order to enable a semi-quantitative evaluation of the given analyte.

In some embodiments of the testing device, the width direction of the testing strip extends at an angle smaller than 90° with respect to a normal of a plane defined by the support structure, i.e., the testing strip is inclined with respect to the plane of the support structure, or in other words, the testing strip width is not parallel to the plane. Also, the testing strip is curved, resulting in an effective extension being shorter than the testing strip center line length in the planar state.

In alternative embodiments, the testing strip center line length is longer than the maximum linear extension of the support structure in the plane.

The liquid sample receiving interface can be a portion of the testing strip and can be configured to receive the liquid sample. Alternatively, the liquid sample receiving interface can also be part of a liquid sample receiving unit. The liquid sample receiving unit, preferably, is arranged on the support structure defining the plane. In some cases, it is advantageous if the liquid sample receiving unit is a separate unit having a liquid sample receiving interface and being connected to the at least one testing strip.

Preferably, the support structure of the testing assembly has maximum linear extension in the plane that is shorter than 5 cm. In some embodiments, the maximal linear extension in the plane is equal to or shorter than 4 cm, preferably shorter than 3 cm and more preferably shorter than 2.5 cm. Preferably, the support structure comprises a hole with a diameter shorter than 4 mm and configured to provide access to the liquid sample receiving interface and thus to allow introduction of a liquid sample. More preferably, the hole is dimensioned and designed to cooperate with a liquid sample providing unit, preferably in the form of a lancet or a needle, for proving the liquid sample to the liquid sample receiving unit.

Preferably, the support structure has a flat or planar geometry that defines the plane. Alternatively, the support structure can be not flat, but an outer perimeter of the support structure defines the plane. In yet another alternative, neither the support structure nor the outer perimeter directly defines a plane and the plane is defined by averaging the spatial position of at least a part of the support structure or of the outer perimeter.

Preferably, the liquid sample receiving unit comprises an absorbent material. The absorbent material, preferably, is configured to be soaked by the liquid sample transferred to the liquid sample receiving unit via the liquid sample receiving interface. The absorbent material can be a porous hydrophilic material, preferably, comprising cellulose, polyesters, modified polyesters, or a similar material such as a micro-structured or a sintered polymer.

The capillary wick of the testing strip can be directly arranged onto the liquid sample receiving interface or in direct contact with the liquid sample receiving unit such that a liquid can be directly transferred from the latter to the former by means of capillary action. Alternatively, the testing strip can be not in direct physical contact with the liquid sample receiving interface or with the liquid sample receiving unit but connected to it through a microfluidic connecting system.

Generally, it is preferred that the testing assembly comprises exactly one testing strip. However, it is also possible that the testing assembly comprises more than one testing strip, in particular, at least two testing strips. The at least two testing strips can be arranged spirally so as to have a respective different projection on the plane defined by the support structure. Alternatively, the testing strips can be arranged on top of each other along a direction perpendicular to the plane defined by the support structure, so that they share a same projection onto the plane. In other words, a total width of the plurality of testing strips arranged on each other corresponds to an addition of each of the testing strip widths of the individual testing strips. Alternatively, both configurations discussed above can be included, i.e., the testing assembly comprises at least two subsets of at least two testing strips, each subset having a different projection onto the plane defined by the support structure the projection shared by all of the testing strips belonging to the subset.

At least one testing strip of the testing assembly can also be arranged so that an angle formed between the width direction and the plane defined by the support structure at each longitudinal position along the longitudinal direction of the testing strip is constant. Thus, the angle between the width direction of the testing strip and the normal of the plane defined by the support structure is constant for every point along the testing strip center line length. This configuration allows for an optimization of the use of the space within the testing assembly. The angle is, in a particular embodiment smaller than 45°. In a preferred embodiment, the angle is lower than 10°. In a more preferred embodiment the angle is lower than 5°. In an embodiment, the angle is 0°. In the latter case, the testing strip is arranged perpendicular (within the limits of fabrication) to a plane defined by the support structure.

Preferably, the capillary wick of at least one testing strip comprises a porous hydrophilic material, preferably comprising cellulose, polyesters, modified polyesters, or a similar material such as a micro-structured or a sintered polymer.

It is advantageous if the testing assembly is configured to enable a transfer of the liquid sample from the liquid sample receiving interface or the liquid sample receiving unit to the test portion along the capillary wick so that every point along a transfer front of the liquid sample reaches the test portion at substantially the same time. The transfer front is to be understood as a time-variable position of an interface differentiating a region of the capillary wick containing liquid sample and a region of the capillary wick not containing liquid sample. In cases where a transfer velocity of the liquid sample is assumed to be constant for every point in the transfer front, the capillary wick of this embodiment is advantageously arranged to interface with the liquid sample receiving interface and the test portion at a first interfacing line and a second interfacing line respectively, so that a length amount of every lateral path between any point along the first interfacing line and the second interfacing line is substantially constant. The term substantially constant is to be understood as a constant value within reasonable limits of fabrication and determination and includes, in some embodiments, length deviations of up to 5%.

The testing assembly can include a conjugate pad that comprises a conjugate material. The conjugate pad is configured to release the conjugate material upon contact with the liquid sample. The reacting material of the test portion can be configured to react in a predetermined manner to a combination of the conjugate material and the liquid sample. This combination is regarded as the specific analyte.

The absorbent material of the liquid sample receiving unit, preferably, is configured to act as a sponge and holds the liquid sample. Once soaked, part of the liquid sample migrates (i.e. is transported by, for instance, capillary action) to the conjugate pad which includes the conjugate material in the form of a so-called conjugate, for example as a dried format of bio-active particles in a salt-sugar matrix configured to guarantee an optimized chemical reaction between a target analyte expected to exist in the liquid sample (e.g., an antigen) and a chemical partner thereof (e.g., antibody). The chemical partner is preferably integrated on the bio-active particle's surface. While the liquid sample dissolves the salt-sugar matrix, it also dissolves the particles. In this way, the target analyte binds to the particles while migrating further through the capillary wick towards the test portion. The test portion of the capillary wick comprises one or more areas (often in the form of stripes) where a reacting material, often in the form of a third molecule is present. By the time the liquid sample-conjugate mix reaches these strips, the target analyte has been bound on the bio active particle from the conjugate pad and the reactive material binds the complex. In reaction thereto, when more and more liquid sample has passed the strips, particles accumulate and the strip changes color. Typically, there are at least two strips in the test portion: a control strip that captures any particle and thereby shows that reaction conditions and technology worked fine, and a second strip that contains a specific capture molecule and only captures those particles onto which an analyte molecule has been immobilized.

The testing assembly can additionally comprise an absorbent pad on a distal end of the testing strip opposite to a proximal end of the testing strip whereto the liquid sample receiving interface is connected. The absorbent pad is configured to stop a black flow of the liquid sample. The absorbent pad is thus configured to act as a sink for the liquid sample, maintaining a flow of the liquid over the capillary wick and preventing a flow of the liquid sample back to or towards the liquid sample receiving unit.

The testing assembly can further comprise at least one solution chamber containing a respective buffersolution, and flow control means configured to control a transfer of the buffer solution to the liquid sample receiving interface or to at least one testing strip. The at least one solution chamber is preferably arranged on the support structure. The solution chamber can be provided as a cavity in the support structure.

Some buffer solutions are advantageously chosen to enhance the transfer of the liquid sample to the test portion. Other buffer solutions comprise a reagent that is configured to react with a particular analyte in a predetermined manner. In case the testing assembly comprises a plurality of solution chambers, different solutions chambers may contain different buffer solutions, which are individually transferred to the liquid sample receiving unit or to a respective testing strip or to a group of testing strips, in accordance with particular requirements of the testing assembly.

The flow control means can be configured to control a transfer of the buffer solution to the liquid sample receiving interface or to the liquid sample receiving unit either before the liquid sample is received via the liquid sample receiving interface, orwhile the liquid sample is being received via the liquid sample receiving interface, or after the liquid sample has been received via the liquid sample receiving interface, or any combination thereof.

The flow control means can alternatively or additionally be configured to control a transfer of the buffersolution to at least one testing strip either before the liquid sample is transferred from the liquid sample receiving interface or the liquid sample receiving unit to the at least one testing strip, or while the liquid sample is being transferred from the liquid sample receiving interface of the liquid sample receiving unit to the at least one testing strip, or after the liquid sample has been transferred from the liquid sample receiving interface or the liquid sample receiving unit to the at least one testing strip, or any combination thereof.

Transferring the buffer solution to the liquid sample receiving interface or to the liquid sample receiving unit or to the capillary wick before the liquid sample is received or transferred respectively causes a wetting of the capillary wick or the absorbent material that in a particular embodiment enhances an absorption capacity.

Transferring the buffer solution to the liquid sample receiving interface or to the liquid sample receiving unit or to the capillary wick while the liquid sample is being received or transferred increases the volume of the liquid present and the flow velocity of the liquid sample and thus reduces the time needed by the liquid sample to reach the test portion of the testing strip.

Transferring the buffer solution to the liquid sample receiving interface or to the liquid sample receiving unit or to the capillary wick after the liquid sample is received or transferred is advantageously used in particular embodiment to wash away the liquid sample towards the test portion.

The flow control means can comprise a soluble material configured to be dissolved in the buffer solution at a predetermined dissolution rate and configured to enable a flow of the buffer solution away from the respective solution chamber after a predetermined time span.

An embodiment comprises a microfluidic system that includes the solution chambers, microfluidic channels for transporting the solution, the body fluid or both the solution and the body fluid, one or more separation chambers, one or more geometric passive valves, one or more waste channels, an inlet connected to the liquid sample receiving interface, one or more outlets connected to the capillary wick, an air vent and an air inlet. Particularly, the microfluidic system is produced by 3D printing, particularly digital light projector 3D printing, on a suitable material and then arranged on the support structure.

In another embodiment, the microfluidic system is embedded in the support structure by surface modification thereof. Advantageously it includes hydrophilic surfaces to enhance capillary flow in some of the microfluidic channels and chambers and hydrophobic surfaces to stop or reduce flow in other microfluidic channels and other sections of the microfluidic system. The hydrophobic surfaces thus act as hydrophobic passive valves.

The microfluidic system is preferably designed to be arranged on or fabricated directly onto the support structure having a maximal extension shorterthan 5 cm, preferably shorterthan 4 cm and more preferably shorter than 2.5 cm.

In a preferred embodiment, the microfluidic system is arranged or fabricated directly onto the support structure, and the optical sensor, the conversion unit, the transmitter unit and, if present, the light source, are arranged on an inner side of the cover unit.

Optionally, the testing assembly can comprise a reservoir containing a soluble material, for instance a pharmacologically inactive substance such as for example lactose monohydrate. This soluble material is configured to be dissolved when in contact with the body fluid. The dissolution of the soluble material is configured to bring in contact a piercing means with the solution chamber. The piercing means, preferably, is configured to pierce the solution chamber and to allow a controlled flow of the buffer solution out of the solution chamber.

The testing assembly can also comprise a lancet, a hollow needle or a catheter or a microfluidic connection system filled with the soluble material. The lancet, hollow needle or catheter are configured and arranged to pierce the solution chamber upon operation (e.g. by applying pressure, or by actuating the testing assembly in a predetermined manner). Once the solution chamber is pierced, the buffer solution enters in contact with the soluble material. Thus, by a proper choice of the soluble material, its amount, and the geometry of the flow control means and the solution chamber, a time span expanding between piercing the solution chamber and the buffer solution reaching the testing strip or the liquid sample receiving unit is controlled.

Optionally, the flow control means can comprise microelectromechanical (MEMS) flow control means which are connected to a power management unit for controlling the transfer of the buffer solution. The microelectromechanical flow control means can include micro-sensors and/or micro actuators, such as micro pumps. The micro sensors and/or micro actuators can be integrated to a microprocessor for controlling micro sensors and/or micro actuators.

The optical sensor and flow control means can be formed by a micro-opto-electro-mechanical system (MOEMS). MOEMS is defined as combination of MEMS merged with Micro-optics. A MOEMS is configured to sense and manipulate optical signals on a very small size scale using integrated mechanical, optical, and electrical systems. A MOEMS includes a wide variety of devices such as, but not limited to optical switches, optical cross-connect, tunable VCSEL, microbolometers etc. These devices are usually fabricated using micro-optics and standard micromachining technologies using materials like silicon, silicon dioxide, silicon nitride and gallium arsenide.

The testing assembly's support structure can have a circular shape with a diameter length shorter than 5 cm. Preferably, the liquid sample receiving interface is arranged at a central position of the support structure. This enables a highly ordered arrangement of the at least one testing strips and thus to facilitate the fabrication of the testing assembly. This is particularly advantageous in testing assemblies with a plurality of testing strips in a spiral configuration.

Alternatively, the liquid sample receiving interface can be arranged away from the central position of the support structure. This is particularly advantageous in testing assemblies wherein two or more testing strips are arranged on each other along the direction perpendicular to the plane. In this particular arrangement, a total width of the plurality of testing strips corresponds to an addition of the individual testing strip widths of each testing strip. For instance, in an exemplary embodiment of the testing assembly, the support structure has an elliptical shape with the liquid sample receiving interface arranged in a position closer to a vertex than to a center of the ellipse. This embodiment is advantageously configured to include longer testing strips than in the case of a circular support structure with the liquid sample receiving unit arranged at the central position.

The testing device can comprise an optional cover unit that is attachable to the support structure.

Alternatively, or additionally, the cover unit of the testing device can comprise an integrated source of light. The source of light can be fed by an electrical power supply unit, for instance a light emitting diode driven by a battery. Alternatively, the integrated source of light comprises a photoluminescent material, preferably a phosphorescent material, wherein radiation absorbed by the material is re-emitted at a lower intensity for up to several hours after the original excitation. In yet another alternative, the testing device may comprise, in addition or as an alternative luminal in a reservoir.

Preferably, the support structure of the testing assembly and the cover unit each are configured such that they are attachable. The testing device can comprise attaching means configured to releasably connect the testing assembly and the cover unit. Suitable attaching means comprise, in a particular embodiment, a bayonet-type attaching structure that comprises at least one peg and a corresponding slot at a respective one of the testing assembly and the cover unit.

The at least one peg can be arranged on the testing assembly, particularly on the support structure, and the corresponding slot, preferably, is arranged on the cover unit. Alternatively, the at least one peg can be arranged on the cover unit and the corresponding slot, preferably, is arranged on the testing assembly, particularly on the support structure of the testing assembly.

In alternative embodiments, the testing device can comprise other attaching means, such as, but not limited to threading elements, snap-lock elements or lock tabs.

Alternatively, the testing device can comprise a cover unit that is non-releasably connected to the testing assembly. In particular, the cover unit may be non-releasably snap-locked to the testing assembly. The optical sensor, and preferably also the conversion unit and the transmitter unit, can be arranged on an inner side of the cover unit, e.g., on a circuit board that is attached to the cover unit.

The testing device can have a substantially fitting of a cylindrical shape, even if the cover unit or the support structure or both the cover unit and the support structure present recessed regions, protruding regions or holes. The bottom base is provided by the support structure and the height is an extension of the cover unit in a direction perpendicular to the support structure. The upper base is also formed by the cover unit. Preferably, the diameter of the base is smaller than 5 cm, more preferably smaller than 3 cm and even more preferably smaller than 2.5 cm. The height of the testing device is preferably between 3 cm and 0.5 cm, more preferably between 2 cm and 1 cm.

The support structure and the cover unit can comprise a thermoplastic polymer suitable for injection molding. The cover unit and the support structure can comprise a respective thermoplastic polymer with different properties. Preferably, the thermoplastic polymer comprised by the cover unit is translucent or transparent.

The testing device can also comprise a testing strip accommodated onto a portion of an inner perimeter of the cover unit. Preferably, the length of the testing strip measured along the testing strip's long axis in a planar state is equal to or smaller than 10 cm, more preferably smaller than 70 mm. The width of the testing strip measured along the testing strip's short axis is preferably equal to or smaller than 6mm, and more preferably smaller than 4 mm. The maximum thickness of the testing strip measured along the axis that is perpendicular to the long and short axis is preferably smaller than 3 mm and more preferably smaller than 2 mm.. If the testing strip has a sample pad and/or a waste pad, the testing strip typically has its maximum thickness in the sections of the sample pad and/or a waste pad.

Preferably, the testing assembly's testing strip is fixed in place so that it cannot move relative to its designated location. In an embodiment, the support structure comprises fixing means for fixing the testing strip thereto.

In some embodiments of the testing device having a cover unit, an inner volume thereof, i.e., the inner volume delimited by the cover unit and by the support structure, comprises a separating structure that divides the inner volume into two sub-chambers by a separation structure. This separating structure thus defines, in particular of these embodiments, a lower chamber and an upper chamber. The lower chamber is delimited by the support structure, a lower portion of the peripheral wall of the cover unit and the separating structure. The upper chamber is delimited by the upper portion of the cover unit and the separating structure. The separating structure comprises at least one opening configured to allow the transport of the liquid sample from the lower chamber to the upper chamber. Preferably, the lower chamber is configured to accommodate a liquid sample providing unit such as, but not limited to, a retractable lancet or a needle, which in a particular embodiment is a hollow needle. Additionally, or alternatively, the lower chamber may accommodate the one or more solution chamber comprising the one or more buffer solutions, as well as the microfluidic systems configured to transport the buffer solution to the designated location within the testing assembly. Also preferably, the upper chamber is advantageously configured to accommodate the testing strips. It is also possible that the testing device comprises a separating structure that is arranged to define more than two sub-chambers.

The testing device can comprise a liquid sample providing module configured to be connected to the liquid sample receiving interface.

The testing device for lateral flow assay, preferably, is modular. In particular, interchangeable liquid sample providing modules may be provided. The testing device can be configured to be used with one specifically designed liquid sample providing module or, alternatively, with various different liquid sample providing modules. This preferred modular nature of the testing device allows the use of a plurality of different testing devices in combination with one single liquid sample providing module.

The liquid sample providing module of the testing device can be or can comprise a needle or a lancet or an array of needles or lancets that are fluidly connected to the liquid sample receiving interface. Alternatively, the liquid sample providing module can be a liquid container which is configured to be fluidly connected to the to the liquid sample receiving interface.

Preferably, the liquid sample providing module comprises at least one piercing element having a tip and a base end, wherein the base end is configured to interface with the liquid sample receiving interface. The piercing element, in an embodiment, can be a needle or a lancet, in particular a blood lancet, having a tip and a base end. Hollow needles further include a channel linking the tip and the base end in fluid communication. In the piercing element, the base end is configured to interface with the liquid sample receiving interface. This allows to extract the liquid sample from a container or living being using the at least one piercing element, and to transfer the liquid sample from the container or living being to the testing strip via the liquid sample receiving interface, that is in some cases integrated in a liquid sample receiving unit and in other cases is part of the testing strip. Alternatively, the liquid sample providing module is in another embodiment a catheter or a cannula.

Upon operation of an exemplary testing device, a liquid sample is transported, preferably by, but not restricted to, capillary action from the tip to the base end of the piercing element. The liquid sample is transported to the testing strip via the liquid sample receiving interface. Once a predetermined amount of liquid sample has reached the liquid sample receiving interface, the liquid sample enters in contact with the one or more testing strips. The predetermined amount of liquid sample depends on a geometry of the liquid sample receiving interface as well as on its physical characteristics, such as the materials comprised, their porosity, etc. The capillary wick of the testing strip enables a transport of the liquid sample, by capillary action, from the liquid sample receiving interface to the test portion, which includes a reactive material configured to react in a predetermined manner to at least one respective analyte, particularly by changing a color of the reactive material when the analyte is in the test portion of the testing strip.

The soluble material that is configured to be dissolved when in contact with the body fluid can be alternatively or additionally configured to activate a detaching mechanism. The detaching mechanism, when activated, moves the liquid sample providing module away from the container or the living being from which the liquid sample is extracted and thus detaches the testing device. The soluble material can comprise a soluble inorganic salt. Alternatively, the soluble material can be a composite of a soluble salt and polymers.

The detaching mechanism can comprise a biased spring attached to the liquid sample proving module. The dissolution of the soluble material in contact with the liquid sample releases the biased spring thus allowing it to return to an unstressed state. This drives the detaching movement. In embodiments where the liquid sample providing module comprises a piercing element, preferably, the dissolution of the soluble material drives a detaching movement of the piercing element that in turn drives the piercing element out of the container or of the living being, thus enabling an end of a liquid sample extraction.

The detaching mechanism can also be integrated in the support structure. For instance, the detaching mechanism can be a portion of the support structure to which the liquid sample receiving interface is arranged. This portion of the support structure is configured to be in a biased state when the soluble material is not yet in contact with the liquid sample. When the soluble material is at least partially dissolved, the portion of the support structure that is in a biased state is allowed to return to an unbiased or unstressed state. This drives the detaching movement. A particular embodiment includes a bi-stable snap dome forming part of the detaching mechanism. Another embodiment comprises a snap dome that is configured to have two or more activated states, each activated state being activated upon application of a respective force amount or in a respective predetermined order. One of the activated states can be configured to cause a piercing of the solution chamber where the buffer solution is stored. Piercing of the solution chamber can also be performed upon activation of other actuators or upon reception of predetermined output signals provided by sensing units comprised by the testing device.

Alternatively, the detaching mechanism can be configured to be directly operable by a user and its operation is not related to the dissolution of the soluble material. For instance, the detaching mechanism can be a mono-stable snap dome triggerthat is activated by applying a predetermined pressure amount. Upon activation, the mono-stable snap dome trigger adopts an unstable state and is configured to return to the stable state after a predetermined time span that depends on the geometry and the material of the snap dome trigger. This snap-dome trigger is in some embodiments configured to drive a retractable liquid sample providing module (for instance a lancet or a needle) in an outward movement configured to start the extraction of the body fluid when the snap-dome trigger is operated by the user and in an inward movement that is configured to end the extraction of the body fluid.

The detaching mechanism can also be implemented as a double push bi-stable actuator. A first push by the user is configured to drive the outward movement of liquid sample providing module (e.g. a lancet or a needle) and a second push by the user is configured to drive the inward movement of the liquid sample providing module, and thus to terminate the extraction of the body fluid. Preferably, a detaching mechanism implemented as a double push bi-stable actuator is configured such that with a first push or with a second push a buffer solution contained in a solution chamber is released from the solution chamber and transferred to the liquid sample receiving interface or to the at least one testing strip. Thus, a detaching mechanism implemented as a double push bi-stable actuator can be configured for automatically releasing buffer solution from a solution chamber upon a first push or upon a second push. For example, detaching mechanism can be configured such that upon a first push or a second push the solution chamber is pierced or cut or sliced by the detaching mechanism.

It is noted that the presence of a liquid sample providing module is not essential. An embodiment of the testing assembly may comprise the soluble material and the detaching mechanism, wherein the detaching mechanism is connectable to an external liquid sample providing module.

A timing function of an extraction of a liquid sample is in some embodiments of the testing device suitably controlled by a kind of the soluble material and its amount and taking into account the nature of the liquid sample. The kind of the soluble material and its amount influences a time span extending between a starting time at which the liquid sample begins to react with the soluble material and an end time at which the detaching movement occurs. The time span can thus be adjusted by choosing the type and the amount of the soluble material.

Additionally, the detaching mechanism can be configured and arranged such that a detaching movement causes a piercing of a container containing the buffer solution. The piercing of the container allows the buffer solution to exit the container. The container is suitably arranged so that the buffer solution, in an exiting movement away from the container, carries the extracted liquid sample together with the dissolved soluble material to the testing strip. Alternatively, or additionally, the buffer solution can be directly provided to the testing strip. Here, the buffer solution is brought in contact with the liquid sample and both travel towards the conjugate pad. The buffer solution, preferably, is configured to chemically react with at least one analyte of the liquid sample in a pre-specified manner.

The at least one of testing strip of the testing assembly is, preferably, arranged so that a shortest distance between two opposite longitudinal ends of the testing strip center line is shorter than the testing strip center line length in the planar state. The spatial disposition of the testing strip within the testing device, preferably, results in a strip having a center line with a center line length that is longer than an effective extension, in any coordinate direction, of the curved testing strip or of the testing strip in a curved state. The center line length is thus a length amount indicative of the testing strip length and thus indicative of a longitudinal extension of the curved testing strip or of the testing strip in a curved state, measured along the center line, und thus takes into account the curvature of the testing strip. The shortest distance is a length amount indicative of a minimum distance amount between a proximal end of the testing strip, i.e., a section of the testing strip being in contact with or in the vicinity of the liquid sample receiving unit, or a section of the testing strip comprising the liquid sample receiving interface, and a distal end of the testing strip at which, or close to which, the test portion is arranged. Thus, providing testing strips with a curved geometry or arranging them in a curved state, or a combination of both, allows from a reduction of a size of the testing device for a given center line length of the testing strip. Preferably, the testing device is configured to have a maximal extension in any spatial direction shorter than 5 cm.

### BRIEF DESCRIPTION OF THE DRAWINGS

In the following, preferred embodiments of the invention are described with reference to the figure. In the figures:
- Fig. 1A:: shows a sectional view of a testing device comprising a piercing element, a testing assembly and a cover unit;
- Fig. 1B:: shows a plan view (top view) of an embodiment of a testing assembly for lateral flow assay;
- Fig. 2A:: shows a schematic representation of a top view and a cross sectional view of a set of four testing strips of a testing assembly for lateral flow assay, the testing strips being in a planar state;
- Fig. 2B:: shows a schematic representation of a top view and a cross sectional view of a set of four testing strips of a testing assembly for lateral flow assay, the testing strips being in a curved state;
- Fig. 3:: shows a schematic representation of an embodiment of a testing assembly for lateral flow assay;
- Fig. 4:: shows a schematic representation of another embodiment of a testing assembly for lateral flow assay that includes a solution chamber and flow control means;
- Fig. 5:: shows a schematic representation of an embodiment of a testing device;
- Fig. 6A:: shows a top view of a testing strip having curved longitudinal edges in planar state;
- Fig. 6B:: shows a lateral view of a testing strip having curved longitudinal edges in a planar state;
- Fig. 7A:: shows an exemplary detaching mechanism in a biased state; and
- Fig. 7B:: shows the detaching mechanism of Fig. 7A in an unstressed state.
- Fig. 8A:: shows a diagram of an exemplary support structure with a microfluidic system arranged thereon.
- Fig. 8B:: shows an enlarged view of a portion of the microfluidic system shown in Fig. 8A.
- Fig. 9:: shows a diagram of an exemplary support structure with a microfluidic system carved thereon.

### DETAILED DESCRIPTION OF THE EMBODIMENTS

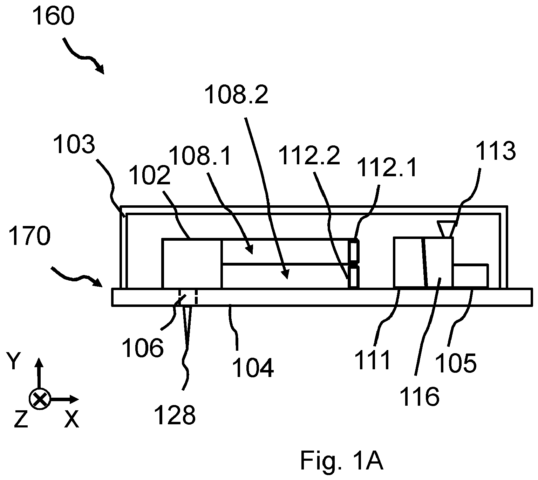

Fig. 1A shows a sectional view of an embodiment of a testing device 160. The testing device 160 comprises a lancet 128. The testing device includes a testing assembly 170 and a cover unit 103. The lancet is a particular and non-limiting example of piercing element of a liquid sample providing module, and is connected to a liquid sample receiving interface 106 of the testing assembly 170. Other suitable liquid sample providing modules include, but are not limited to, needles, hollow needles or cannulas The testing assembly further comprises a support structure 104, a liquid sample receiving unit 102 and two testing strips 108.1 and 108.2, each comprising a respective test portion 112.1 and 112.2.

The testing device 160 also comprises a power management unit 105 comprising voltage stabilizing circuitry. The testing device further comprises an optical sensor 111 that is configured for detecting impeding light that is reflected by the test portions 112.1 and 112.2, and for converting the detected light into an electrical signal representing the intensity and/or the color of the impeding light. The optical sensor 111 is connected to a conversion unit 116. The conversion unit is configured for converting an electrical signal into digital data representing an intensity and/or a color of the detected light. The conversion unit 116 is an analog-to-digital converter and is comprised by the optical sensor 111. Alternatively, the conversion unit can be a separate component that is arranged on the support structure 104 and operatively connected to the optical sensor 111. For example, the optical sensor 111, the power management unit 105 and the transmitter unit having an RF-interface can be arranged on the support structure 104. Light reflected from the test portions 112.1 and 112.2 can be directed to the optical sensor using one or more mirrors also arranged on the support structure 104. Using mirrors, a light path can be created linking the test portions 112.1 and 112.2 and the optical sensor 111. It is also possible that the optical sensor 111, the power management unit 105 and the transmitter unit having an RF-interface are arranged on a circuit board, e.g., a flexible PCB. The circuit board can be arranged on the support structure 104. Using optical elements such as mirrors a light path can be created from the test portions 112.1 and 112.2 to the optical sensor 111 that is arranged on the circuit board. The circuit board can also be attached to the inner surface of the cover unit 103, the inner surface facing the support structure. Preferably, the optical sensor is arranged such that if the circuit board is attached to the inner surface of the cover unit 104, the optical sensor likewise faces the support structure. Since the test portions 112.1 and 112.2 face the sidewalls of the testing device, preferably, an optical element is arranged and configured to redirect light reflected from test portions 112.1 and 112.2 about 90° towards the optical sensor 111. Further comprised can be one or more light sources, e.g., LEDs, that are arranged and configured for illuminating test portions 112.1 and 112.2. The one or more light sources can be arranged on the support structure 104, or on a circuit board, or directly to the inner surface of the cover unit 103.

The testing device 160 also comprises a transmitter unit 113 connected to a power management unit 105 and to the conversion unit 116. The transmitter unit 113 is configured to wirelessly transmit digital data representing the intensity and/or color of the detected light, e.g., in accordance with a predetermined wireless communication protocol.

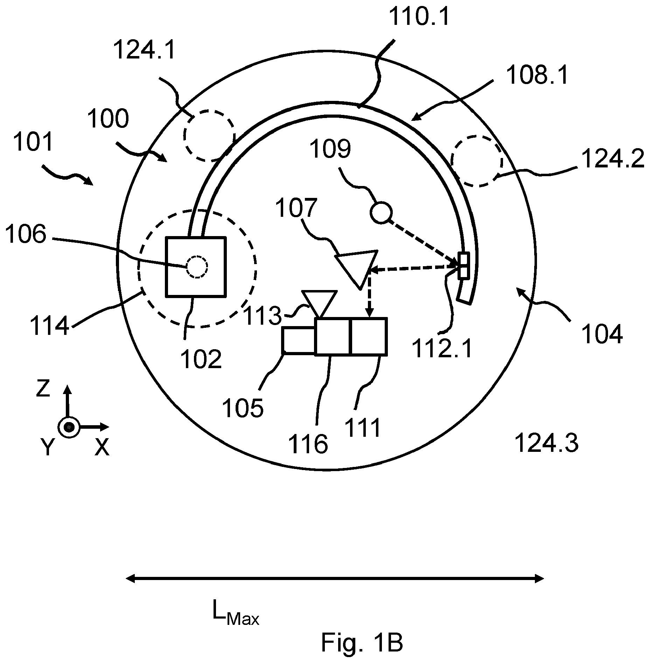

Fig. 1B shows a cross plan view (top view) of an exemplary embodiment of a testing device 101 having a testing assembly 100 for lateral flow assay. In the following discussion, those features being shared by the testing device 160 of Fig. 1A and 101 of Fig.1B, are referred to using the same numerals.

The testing device 101 of Fig. 1B comprises a liquid sample receiving unit 102 that is arranged on a support structure 104. In alternative and preferred testing devices, the liquid sample receiving unit is arranged on a central position of the support. In other testing devices (not shown), the liquid sample receiving interface can be arranged directly on the testing strip, and thus, these alternative testing devices do not have a dedicated liquid sample receiving unit, as testing device 101 does. The support structure 104 is a flat structure that defines a plane XY as defined by the axes shown in Figs. 1A, and 1B. The support structure 104 has a largest linear extension L_{Max} that is shorter than 5 cm, preferably shorter than 3 cm, and more preferably 2.5 cm . The liquid sample receiving unit 102 comprises a liquid-sample receiving interface 106 in the form of an opening on the support structure 104. The liquid sample receiving unit 102 is configured to receive a liquid sample via the liquid sample receiving interface 106. The liquid sample receiving unit includes an absorbent material (not shown), preferably a porous hydrophilic material, preferably comprising nitrocellulose or a similar material.

The testing assembly 100 also includes two testing strips 108.1 and 108.2. Each testing strip 108.1, 108.2 is fluidly connected to the liquid sample receiving unit 102 and each comprises a capillary wick (110.1, 110.2) connected to the liquid sample receiving unit 102. Preferably, the capillary wick also comprises a porous hydrophilic material such as nitrocellulose or a similar material. Each testing strip 108.1, 108.2 includes a respective test portion 112.1, 112.2 as part of the capillary wicks 110.1 and 110.2. The test portions include a respective reacting material (not shown) configured to react in a predetermined manner to at least one respective analyte. In some testing assemblies, the test portions 112.1 and 112.2 may contain different reacting materials configured to react to different analytes. In other testing assemblies, the test portions comprise a single reacting material configured to react to a given analyte, with a same or a respective different sensitivity, in orderto either improve the accuracy of testing assembly or in order to enable a semi-quantitative evaluation of the given analyte. Other alternative testing assemblies may comprise a plurality of test portions having a given material and additionally at least one test portion having a different reacting material.

In this particular testing assembly 100, the two testing strips 108.1 and 108.2 are arranged so that an angle formed between a width direction of the testing strip (Z, in the particular embodiment of Figs. 1A, and 1B and the normal N of the plane (XY) at each longitudinal position along the longitudinal direction of the testing strip is substantially constant with an angle value of substantially 0°, within the practical limits of fabrication and angle determination. This means that the width direction of the testing strip is perpendicular to the support structure 104.

Additionally, a testing assembly can further comprise a first window section 114 (dashed line) arranged around the liquid sample receiving unit 102. The first window section 114 is at least partially transparent in a visible wavelength range and is arranged to allow a control of a positioning of the liquid sample receiving unit onto an external surface. By enabling a userto partially see an external surface onto which the testing assembly is to be positioned, the exact position of the liquid sample receiving unit can be advantageously controlled.

The testing device 101 also comprises a power management unit 105 having an energy storage unit for storing electric energy and for providing electrical power, and an optical sensor 111. The optical sensor 111 is configured and arranged for detecting impeding light that is reflected from the test portions 112.1 and 112.2. The optical sensor 111 is configured to convert the detected light into an electrical signal representing the intensity or color of the impeding light.

The testing device 101 of Fig. 1B also includes a light source 109 that is arranged and configured to illuminate the test portion 112.1. Further, an optical element 107 is arranged and configured for directing light provided by the light source 109 and reflected from the test portion 112.1, to the optical sensor 111. Preferably, the optical sensor 111, the conversion unit 116 and the transmitter unit 113 are arranged on an inner side of a cover unit of the testing device (not shown). Also, the light source 109 and the optical member are preferably arranged on the inner side of the cover unit. Alternatively, the optical sensor 111, the conversion unit 116 and the transmitter unit 113 can be arranged on the support structure 104 or on a circuit board that is arranged on the support structure 104. It is also possible to arrange at least one of the optical sensor 111, the conversion unit 116 and the transmitter unit 113 on an outer side of a cover unit of the testing device.

The optical sensor 111 is operatively connected to the conversion unit 116 that is configured for converting an electrical signal into digital data. The transmitter unit 113 is part of a transceiver unit 117 connected to the power management unit 105 and to the conversion unit 116.

Via a data bus, the conversion unit 116 is connected to the transceiver unit 117 for providing digital data to the transceiver unit's transmitter unit 113.

The transceiver unit 117 is configured to receive control commands and to transmit digital data in accordance with a predetermined wireless communication protocol. The transceiver unit 117 is configured for drawing energy from an external device. The transceiver unit 117 thus has energy harvesting capabilities and is configured for drawing energy from an external device via electromagnetic induction. To this end, the transceiver unit's transmitter unit comprises a NFC-coil and an NFC-chip for near-field- communication with the external device. The power management unit 105 comprises a capacitor, preferably, a supercapacitor, for storing harvested energy. In alternative embodiments no energy storage unit is present and the harvested energy is directly used for powering components of the testing device 101.

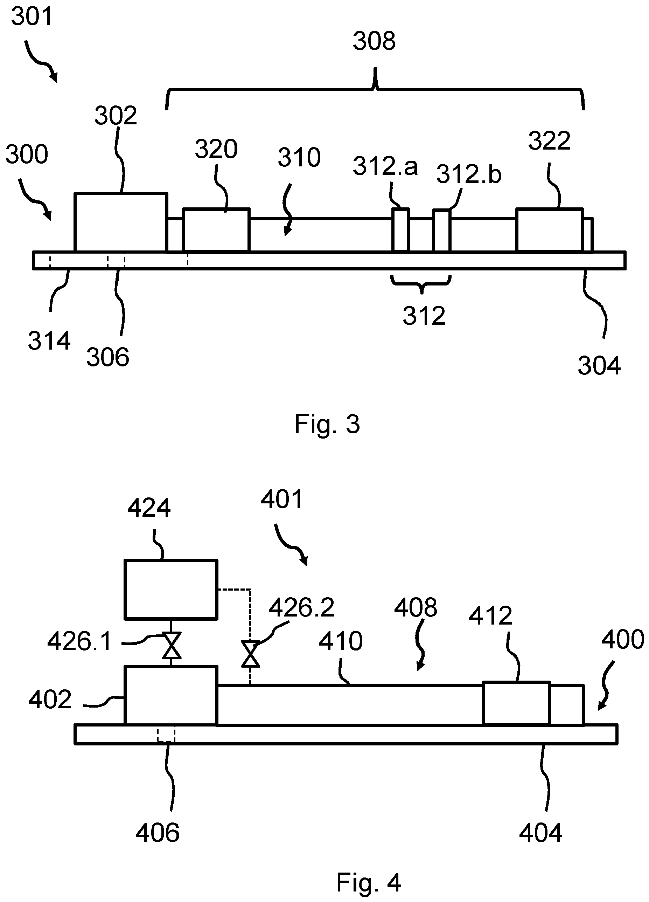

Alternatively, or additionally, the testing device 101 can comprise one or more solution chambers 124.1, 124.2 (dashed lines) that contain a respective buffer solution. The testing devices 101 can also include an optional flow control means (not shown in Fig. 1, see description of Fig. 4) that is advantageously configured to control a transfer of the buffer solution to the testing strips 108.1, 108. 2. In some testing assemblies each solution chamber is connected to every testing strip. In alternative testing assemblies, however, some solution chambers are only connected to only one or to a sub-set of the testing strips.

In some embodiments of the testing device (not shown), the flow control means may be configured to control a transfer of the solution buffer to the liquid sample receiving interface or to the liquid sample receiving unit. In some testing devices comprising two or more solution chambers, at least one of the solution chambers is connected to the liquid sample receiving interface and at least one of the solution chambers is connected at least one of the testing strips.

In any of the previously described testing devices, the capillary wick of the testing strip may be arranged on a testing-strip carrier that is configured to confine at least a part of incoming light inside a light-guiding layer of the carrier by total internal reflection achieved, for instance, by a proper choice of materials with a suitable respective refractive index or position-dependent refractive index profile. The testing-strip carriers also comprise a light output section onto which the test portion of the testing strip is suitably arranged. The light output section is configured to enable confined light to exit the testing-strip carrier. Therefore, these particular testing-strip carriers are suitably configured to illuminate the test portion arranged thereon from its rear part. Advantageously, in some embodiments, the capillary wick has a thickness that is thin enough to let at least part of the light impinging on the rear part of the test portion to travel to the front part.

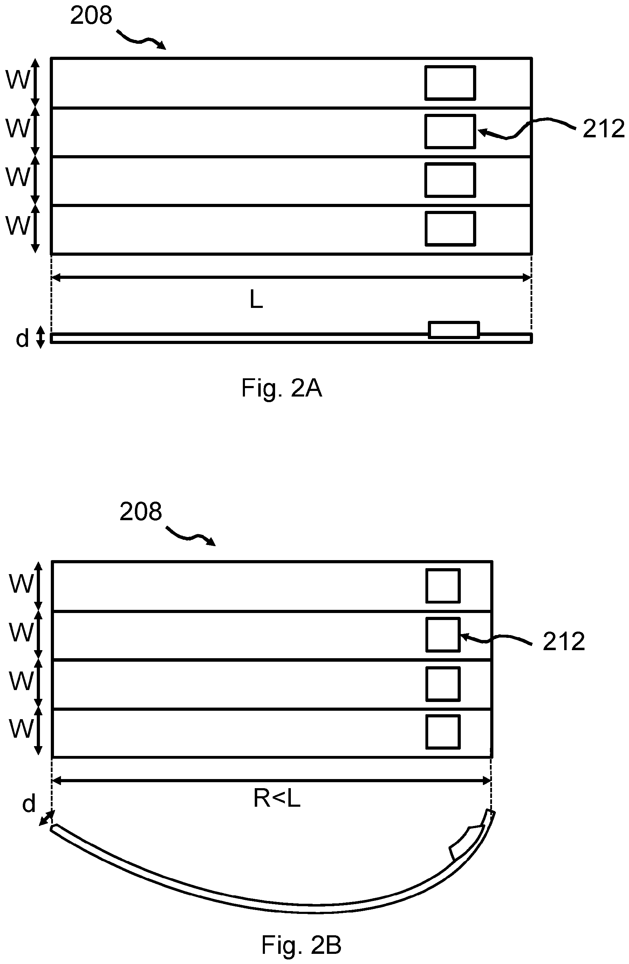

The geometry of an exemplary set of testing strips 208 is described with reference to Figs. 2A and 2B. In Fig. 2A, four testing strips form a set of testing strips. Each individual testing strip has a respective test portion 212. Each testing strip is presented in an planar state and has a testing strip center line length L in a longitudinal direction, a testing strip width W in a width direction perpendicular to the longitudinal direction and a testing strip thickness d, in a thickness direction perpendicular to both the longitudinal direction and the width direction, that is shorter, i.e., has a smaller extension than the testing strip center line length L and the testing strip width W. Fig. 2B shows the same set of testing strips 208 in a curved state in which a shortest distance between two opposite longitudinal ends of the testing strip center line, or in other words, an effective extension R is shorter than the testing strip center line length L in the planar state shown in Fig. 2A. In this particular example, the shortest distance between the two opposite longitudinal ends of the testing strip corresponds to the effective extension R. In another exemplary configuration (not shown) wherein the testing strip is bent in e.g. a circular shape, the shortest distance between the two opposite longitudinal ends vanishes, whereas the effective extension corresponds to the diameter of the formed circle, which is *π*/*L.* In any case, the shortest distance and the effective extension are shorter than the testing strip center line length.

Fig. 3 shows a schematic representation of another embodiment of a testing device 301 having a testing assembly 300. The testing assembly 300 shares many features with the testing assembly 100 described with respect to Fig. 1B. Those features shared will be referred to by using the same reference numbers, only altering the first digit, which is "1" when referring to Fig. 1 and "3" when referring to Fig. 3.

The testing assembly 300 comprises a support structure 304 that has an opening 306 which, in this particular testing assembly is in connection with a liquid sample receiving unit 302. In alternative embodiments of the testing assembly, the opening is directly connected to a section of the testing strip acting as a liquid sample receiving interface. The liquid sample receiving interface is advantageously configured to interface with an external liquid sample providing module (not shown). Liquid sample providing modules that can be connected to the liquid sample receiving interface 306 may include, for example, lancets, needles, cannulas or liquid containers with means to transfer a liquid sample contained therein to the liquid sample receiving unit 302 via the liquid sample receiving interface 306. Alternatively, the liquid sample can be directly supplied to the liquid sample receiving interface without the need of a liquid sample providing module.

The testing assembly 300 includes one testing strip 308 in a curved state (nor shown) that is fluidly connected to the liquid sample receiving unit 320. The testing strip 308 comprises a capillary wick 310. The testing strip also includes conjugate pad 320 that comprises an immobilized conjugate material. The conjugate pad 320 is configured to release the immobilized conjugate material upon contact with the liquid sample. The conjugate material is contained in the conjugate pads, i.e. as colloidal gold, or colored, fluorescent or paramagnetic monodisperse latex particles that have been conjugated to one specific biological component expected to be identified in the liquid sample. This biological component is in some testing devices an antigen and in other testing devices an antibody. The testing strip 308 also comprises test portion 312 that includes a test line 312.1 and a control line 312.2 forming a so-called reaction matrix.

The liquid sample, received through the liquid sample receiving interface 306 is transported by capillary action from the liquid sample receiving unit 302 along the capillary wick 310. At the conjugated pad 320, the liquid sample releases the conjugate material and a combination of both is further transported towards an absorbent pad 322 located at a distal end of the testing strip 308, opposite to a proximal end whereto the liquid sample receiving unit 302 is connected. The absorbent pad 322 of this (and similar) testing strips is configured to act as a sink for the liquid sample, maintaining a flow of the liquid over the capillary wick and preventing a flow of the liquid sample back to or towards the liquid sample receiving unit 302.

The testing device 301 also comprises an optical sensor (not shown) that is arranged and configured for detecting light reflected from the test portion 312 of test strip 308. The optical sensor is further configured for converting impeding light into an electrical signal representing the intensity and/or the color of the detected light. The testing device 301 comprises a conversion unit for converting an electrical signal into digital data and a transmitter unit for wirelessly transmitting digital data upon initiating by an external initiator device.

The features distinguishing the testing assembly 300 from testing assembly 100 can be advantageously used in combination with any of the alternatives to the testing device 100 that have been previously discussed. For instance, some testing devices may include, in addition to the features discussed with reference to Fig. 3, a reflector element or at least one solution chamber with respective flow control means, or, preferably, both a reflector element and at least one solution chamber with respective flow control means. Some of these testing assemblies also comprise a testing-strip carrier onto which the capillary wick is arranged.

Fig. 4 shows a schematic representation of another embodiment of a testing device 401 testing assembly 400. Here again, the testing assembly 400 shares some features with the testing assemblies 100 and 300 described with respect to Figs. 1B, and 3. Those features shared are referred to by using the same reference numbers, only altering the first digit, which is "1" when referring to Fig. 1, "3" when referring to Fig. 3 and "4" when referring to Fig. 4.

The testing device 401 comprises a solution chamber 424 containing a buffer solution, and flow control means 426.1 configured to control a transfer of the buffer solution to the liquid sample receiving unit 402. Alternatively, or additionally, some testing devices include flow control means 426.2 that control a transfer of the buffer solution directly to the testing strip 408 (as indicated by the dashed-line). Some testing devices include a plurality of solution chambers and control flow means that control a respective transfer of the respective solution (which can be identical or different or a combination thereof) to the liquid sample receiving interface or to one or more testing strips. Buffer solutions are advantageously chosen to enhance a transport of the liquid sample along the capillary wick of the testing strips.

The flow control means 426.1 and 426.2 preferably comprise microelectromechanical (MEMS) flow control means for controlling the transfer of the buffer solution. The microelectromechanical flow control means preferably is connected via a data bus to a microcontroller. Via a transceiver unit control commands can be received for controlling the microelectromechanical flow control means by way of the microcontroller. The microelectromechanical flow control means include, in different testing assemblies, micro-sensors and/or micro actuators, such as micro pumps. In a particular testing device, the micro sensors and/or micro actuators are also integrated to a microprocessor for controlling micro sensors and/or micro actuators.

The testing device 401 comprises an optical sensor (not shown) for detecting light that was reflected from the test portion 412 of test strip 408, and for converting the light into an electrical signal representing the light intensity and/or color. The testing device 401 comprises a conversion unit (not shown) for converting an electrical signal into digital data and a transmitter unit (not shown) operatively connected to the conversion unit for transmitting digital data to an external receiving device, e.g., via a near-field communication (NFC) link. The transmitter unit can comprise an NFC-chip or a RFID-tag. Alternatively, the transmitter unit can be configured for transmitting the digital data via Bluetooth or Wi-Fi.

With energy drawn from the external device, electronic and/or electro-mechanical components of the testing device can be powered.

In particular, energy supply for such micro-pumps or micro actuators preferably is wireless, for instance when reading out the transmitter unit via NFC link.

The capillary wick of some of the testing assemblies is arranged on a testing-strip carrier configured to confine by internal total refection at least a part of incoming light inside a light-guiding layer of the carrier. The test portion of the testing strip is arranged onto a light output section of the testing carrier, so that light confined inside the light-guiding layer can exit it and thereby illuminate the test portion.

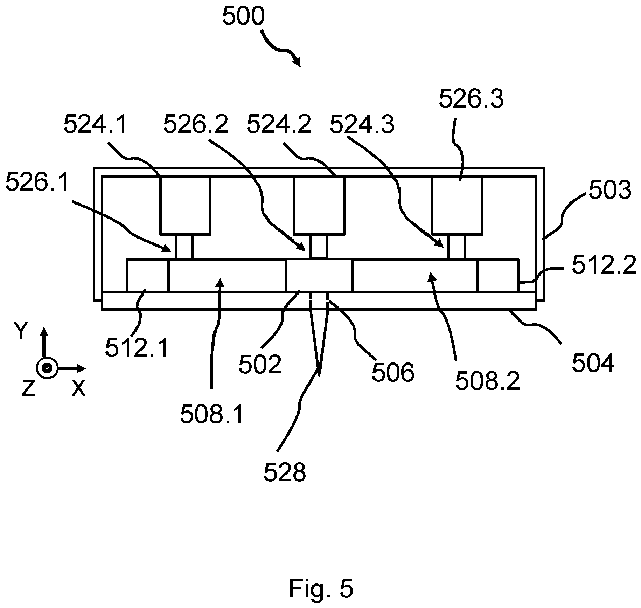

Any of the testing assemblies described in the previous discussion can form part of a testing device as described with reference to Fig. 5.

Fig. 5 shows a schematic representation of an embodiment of a testing device 500 for lateral flow assay. The testing device 500 comprises a liquid sample providing module in the form of a lancet 528 that is configured to be connected to the liquid sample receiving interface 506 of the liquid sample receiving unit 502. Here again, the testing device 500 comprises a testing assembly that shares features with the testing assemblies 100 and 400 described with reference to Figs. 1 and 4. These features share the same reference numbers except for the first digit, which is "1" when referring to Fig. 1, "4" when referring to Fig. 4 and "8" when referring to Fig. 5.

The testing device 500 comprises three distinct solution chambers 524.1, 524.2 and 524.3. It also comprises flow control means that include microelectromechanical flow means 526.1, 526.2, 526.3 configured control flow of the buffer solution to the testing strips 510.1, 510.2 or to the liquid-sample receiving unit 502.

In some embodiments of the testing device, the testing device includes flow control means that are alternatively or additionally configured to control the transfer of the buffer solution while the liquid sample is being transferred to the liquid sample receiving interface via the liquid sample providing module.

Yet other testing devices can include flow control means that are alternatively or additionally configured to control the transfer of the buffer solution after the liquid sample has been transferred to the liquid sample receiving interface via piercing element.

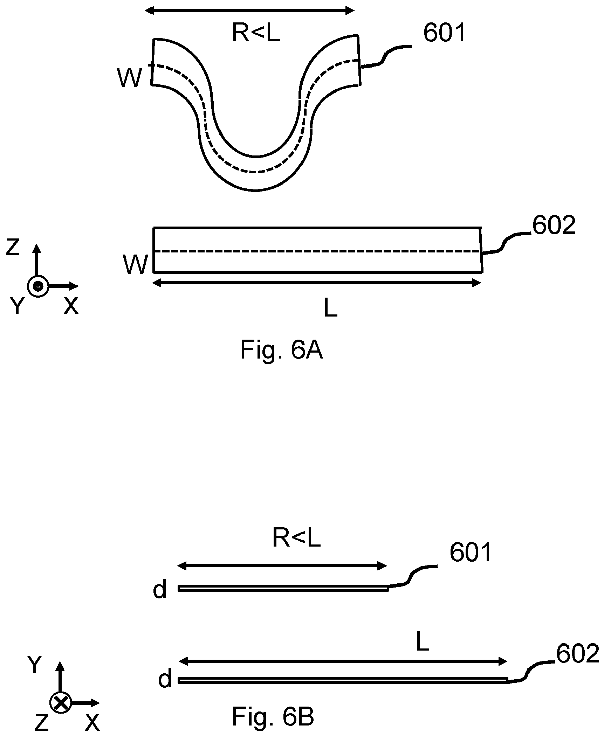

Fig. 6A shows a top view of a testing strip 601 in an alternative geometrical configuration that is used in some embodiments of the testing assemblies described with reference to Figs. 1A, 1B, 3 and 4. Fig. 6A shows top views of a testing strip 601 of width W, with curved longitudinal edges and a testing strip center line length L given by the length measure of the center line (dashed lined) and a testing strip 602 with straight longitudinal edges, that has the same width W and the same testing strip center line length L as the testing strip 601). Fig 6B shows a corresponding lateral view of the testing strips 601 and 602. The thickness of the testing width is given by d.

The testing strip 601 has already in the planar state an effective extension R that is shorter than the maximal longitudinal extension L of the testing strip in the planar state. The effective extension of the testing strip length in the planar state is in the case depicted in Fig. 6A equivalent to the testing strip center line length (dashed line). In orderto achieve, for testing strip 602, an effective extension shorter than L, the testing strip 602 has to be arranged in a curved state, e.g. by folding, curving, wrapping, etc. the testing strip 602.

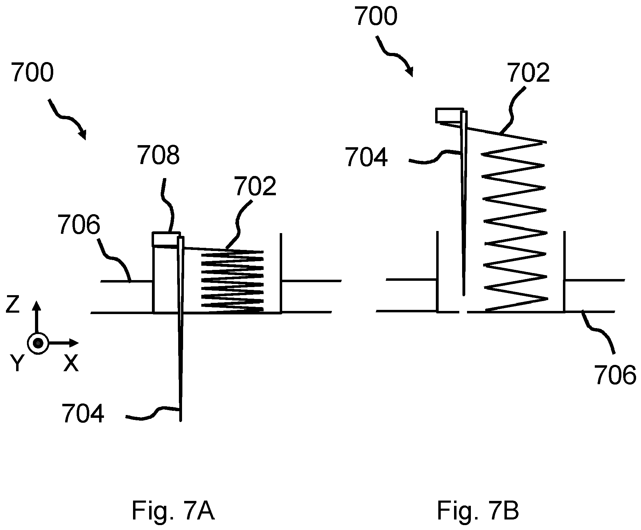

Figs. 7A and 7B show an exemplary detaching mechanism 700 that can be used in combination with any of the testing devices described hereinabove. Fig. 7A shows the detaching mechanism 700 having a spring 702 in a biased state, wherein Fig. 7B shows the same detaching mechanism 700 having the spring 702 in an unstressed or unbiased state. A distal end of the spring 702 is connected to a lancet 704 that forms in this particular case the liquid sample providing module of the testing device. Other detaching mechanisms in accordance with this invention can be alternatively attached to other liquid sample providing modules such as flexible catheters or other fluidic systems. A proximal end of the spring 702 is connected to the support structure 706 of a testing device at an anchor point. The lancet 704 is also in fluid communication with a soluble material 708 that is configured to remain attached to the support structure as long as a predetermined fraction of the soluble material remains in a solid state. When liquid enters in contact with the soluble material, it causes a dissolution thereof that enables a detachment of the spring 702 from the support structure 706. The spring is thus allowed to adopt an unbiased state as shown in Fig. 7B, forcing a movement of the lancet 704 in a Z direction. This detaching movement drives the lancet from the container or the living being from which it was extracting the liquid sample into an inner volume of the testing device. This detaching movement is configured to end an ongoing liquid sample extraction process. Other detaching mechanisms that can be used alternatively may comprise a bi-stable snap dome, connected to the liquid sample providing unit and wherein a transition from a first stable state to a second stable state is driven by a dissolution of at least a fraction of the soluble material.

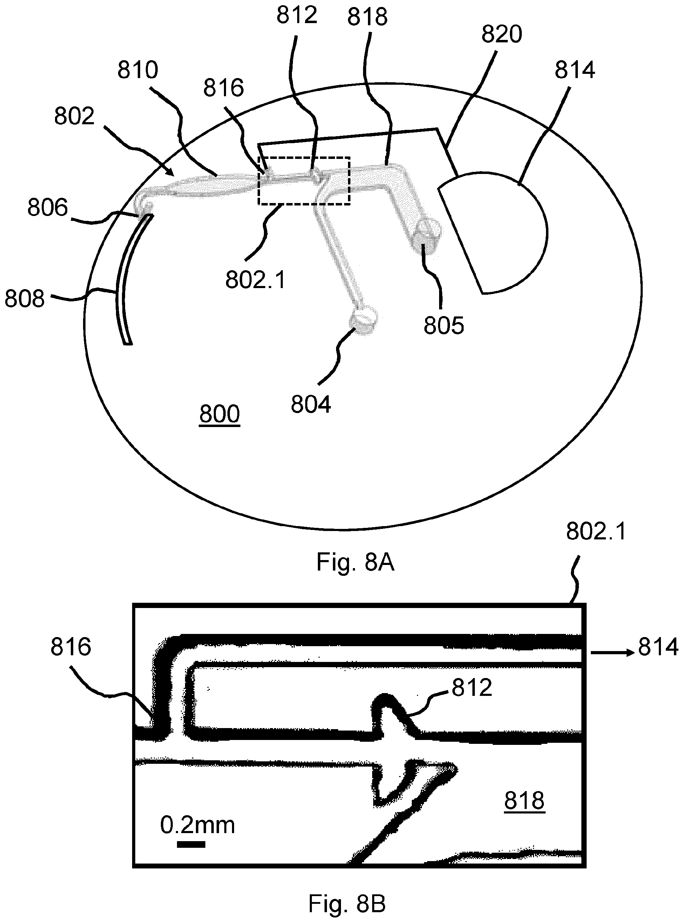

Figure 8A shows a diagram of a support structure 800 with a passive microfluidic system 802 arranged thereon Figure 8B shows an enlarged view of a portion 802.1 of the microfluidic system shown in Fig. 8A. The microfluidic system comprises 802 an inlet 804 connected to the liquid sample receiving interface for receiving the liquid sample. The microfluidic system also comprises an outlet connected to the testing strip 808, of which only a section is shown in Fig. 8. The inlet 804 is connected to an air vent 805 via a waste channel 818. Further, the inlet 805 and waste channel are fluidly connected to the outlet 806 via a passive valve 812 and a separation chamber 810. An air reservoir 814 is connected via a dedicated connection 820 to an air inlet 816 arranged between the passive valve 812 and the separation chamber. The passive microfluidic system 802 with geometric passive valves can be produced separately from the support structure 800 and then arranged onto it. It can also be connected to a container containing a buffer solution (nor shown). Suitable fabrication methods for the microfluidic system 802 include 3D printing, in particular digital light projector 3D printing (DLP 3D printing).

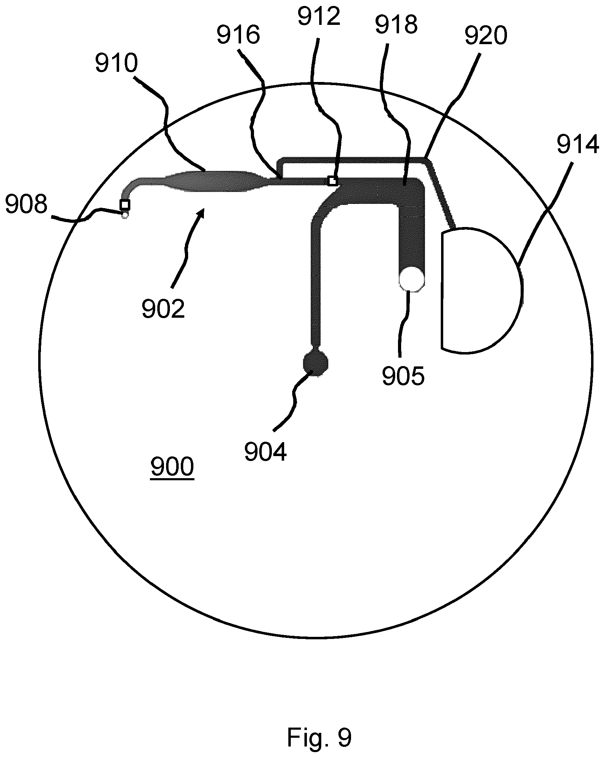

Fig. 9:shows a diagram of an exemplary support structure 900 with a microfluidic system 902 carved thereon. The features corresponding to those features of Figs. 8A and 8B are referred to using the same numerals except forthe first digit, which is "8" forthe microfluidic system of Figs. 8A and 8B and "9" for the microfluidic system of Fig. 9. The passive valve 912 is, in a particular exemplary microfluidic system, not a geometric passive valve as valve 812, but a hydrophobic valve, i.e. a portion of the microfluidic system coated with a hydrophobic surface, particularly a nano-coating, to limit the flow of a liquid. A similar hydrophobic surface is also located in the immediate vicinity of the outlet 908, as indicated by the white box in Fig. 9. Also, the dedicated connection 920 between the air reservoir 914 and the air inlet 916 is optionally coated with a hydrophobic surface. Preferably, the remaining surfaces, including the waste channel 918, the connection linking the waste channel with the inlet 902, and the separation chamber are coated with a hydrophilic material forming hydrophilic surfaces suitable for enhancing capillary flow in the respective sections of the microfluidic system 902.

In summary, the invention relates to a device having a testing assembly for lateral flow assay. The testing assembly comprises liquid sample receiving interface arranged on a support structure defining a plane. The liquid sample receiving interface is configured to receive a liquid sample. The testing assembly comprises at least one testing strip fluidly connected to the liquid sample receiving interface. The testing strip comprises a capillary wick fluidly connected to the liquid sample receiving interface and including at least one test portion, the test portion comprising at least one respective reacting material configured for reacting in a predetermined manner to at least one respective analyte. The testing device further comprises an optical sensor that is arranged and configured for detecting impeding light that was reflected from the at least one test portion, and for converting the detected light into an electrical signal representing the light intensity or the color. The testing device also comprises a conversion unit for converting an electrical signal into digital data. The testing device also comprises a transmitter unit for wirelessly transmitting digital data representing the intensity and/or the color of the detected light to an external device.

## Claims

1. A testing device (160) comprising a testing assembly (170) for lateral flow assay, the testing assembly comprising:
- a liquid sample receiving interface (106) arranged on a support structure (104) defining a plane (XY), the liquid sample receiving interface being configured to receive a liquid sample,
- at least one testing strip (108.1, 108.2) fluidly connected to the liquid sample receiving interface, the testing strip comprising
- a capillary wick (110.1, 110.2) fluidly connected to the liquid sample receiving interface and including at least one test portion (112.1, 112.2), the test portion comprising at least one reacting material configured for reacting in a predetermined manner to at least one specific analyte,
wherein the testing device (160) further comprises
- an optical sensor (111), arranged and configured for detecting light reflected from the at least one test portion and for converting the detected light into an electrical signal representing an intensity and/or a color of the detected light,
- a conversion unit (116) for converting the electrical signal into digital data representing the intensity and/or the color of the detected light, and
- a transmitter unit (113) for wirelessly transmitting digital data.

2. The testing device (160) of claim 1, wherein the transmitter unit is configured for transmitting the digital data via a near-field communication link.

3. The testing device (101) of claim 1 or 2, comprising at least one optical element (107) that is arranged and configured for directing light reflected from the at least one test portion to the optical sensor.

4. The testing device (101) of any one of the preceding claims, comprising at least one light source (109) that is arranged and configured to illuminate the at least one test portion.

5. The testing device (160) of claim 4, comprising a further optical element that is arranged and configured for directing illuminating light emitted by the at least one light source to the at least one test portion.

6. The testing device (160) of at least one of the preceding claims, wherein the testing assembly further comprising at least one solution chamber (424) containing a respective buffer solution, and flow control means (426) configured to control a transfer of the buffer solution to the liquid sample receiving interface (402) or to the at least one testing strip (408).

7. The testing device (160) of claim 6, wherein the flow control means (426) is configured to control a transfer of the buffer solution from the solution chamber (424) to the liquid sample receiving interface (402) either:
- before the liquid sample is received via the liquid sample receiving interface (406); or
- while the liquid sample is being received via the liquid sample receiving interface; or
- after the liquid sample has been received via the liquid sample receiving interface; or
- any combination thereof.

8. The testing device (160) of claims 6 or 7, wherein the flow control means is configured to control a transfer of the buffer solution from the solution chamber (424) to the at least one testing strip (408) either:
- before the liquid sample is transferred from the liquid sample receiving interface (406) to the at least one testing strip (408); or
- while the liquid sample is being transferred from the liquid sample receiving interface to the at least one testing strip; or
- after the liquid sample has been transferred from the liquid sample receiving interface to the at least one testing strip; or
- any combination thereof.

9. The testing device (160) of at least one of the claims 6 to 8, wherein the flow control means comprises microelectromechanical flow control means which are connected to the power management unit for controlling the transfer of the buffer solution.

10. A testing device (160) of at least one of the claims 1 to 9, wherein testing device (160) further comprises a cover unit (103) attachable to the support structure.

11. The testing device (160) claim 10, wherein the testing assembly is non-releasably connected to the cover unit.

12. The testing device (160) of at least one of the preceding claims, comprising a liquid sample providing module (528), the liquid sample providing module comprising at least one piercing element or a cannula (530) having a tip and a base end, wherein the base end is configured to interface with the liquid sample receiving interface.

13. A testing system for testing a liquid sample for the presence of a specific analyte, the testing system comprising
- a testing device according to at least one of the preceding claims, and
- an external device that is configured for receiving digital data provided by the testing device.

14. The testing system of claim 1, further comprising a server that is operatively connected to the external device for transmitting digital data received by the external device to the server.
